Europäisches Patentamt

European Patent Office

Office européen des brevets

(19)

(11) EP 0 324 809 B1

(12) **EUROPÄISCHE PATENTSCHRIFT**

(45) Veröffentlichungstag und Bekanntmachung des Hinweises auf die Patenterteilung:
28.08.1996 Patentblatt 1996/35

(21) Anmeldenummer: 88905657.8

(22) Anmeldetag: 27.06.1988

(51) Int Cl.6: **G01N 27/00**, G01N 27/72

(86) Internationale Anmeldenummer:
PCT/DE88/00383

(87) Internationale Veröffentlichungsnummer:
WO 88/10420 (29.12.1988 Gazette 1988/28)

(54) **VERFAHREN ZUR ERFASSUNG DES RANDSCHICHTZUSTANDES VON KÖRPERN**

PROCESS FOR DETERMINING THE EDGE LAYER CONDITION OF OBJECTS

PROCEDE POUR DETECTER L'ETAT DE LA COUCHE SUPERFICIELLE DE CORPS

(84) Benannte Vertragsstaaten:
CH DE FR GB IT LI

(30) Priorität: 27.06.1987 DE 3721254

(43) Veröffentlichungstag der Anmeldung:
26.07.1989 Patentblatt 1989/30

(73) Patentinhaber: STIFTUNG INSTITUT FÜR WERKSTOFFTECHNIK
D-28717 Bremen (DE)

(72) Erfinder: KLÜMPER-WESTKAMP, Heinrich
D-2820 Bremen 77 (DE)

(74) Vertreter: Möller, Friedrich, Dipl.-Ing. et al
Meissner, Bolte & Partner
Patentanwälte
Hollerallee 73
28209 Bremen (DE)

(56) Entgegenhaltungen:
EP-A- 0 100 009     AT-A- 355 838
DE-A- 3 439 061     FR-A- 2 590 671
GB-A- 2 161 938     GB-A- 2 184 553
US-A- 4 332 833

- Härterei-Technische Mitteilungen, Band 41, Nr. 4, Juli/August 1986, Carl Hanser Verlag, (München, DE), R. Conrad et al.: "Zerstörungsfreie Ermittlung von wärmebeeinflussten Randschichten und deren Dicke", Seiten 213-218
- Patent Abstracts of Japan, Band 9, Nr. 37 (P-335) (1760), 16 Februar 1985; & JP-A-59178356 (NIPPON DENSHI K.K.) 9 Oktober 1984

**Beschreibung**

Die Erfindung betrifft ein Verfahren zur Erfassung des Randschichtzustandes von Körpern. Weiterhin betrifft die Erfindung die Verwendung dieses Verfahrens.

Das Nitrieren wie auch das Nitrocarburieren stellen neben dem Aufkohlen zur Zeit die wichtigsten und am meisten verbreiteten Verfahren der thermochemischen Randschicht-Behandlung dar. Diese werden angewendet, um die Eigenschaften von Bauteilen und Werkzeugen hinsichtlich Verschleiß, Ermüdung und Korrosionswiderstand entsprechend den an sie gestellten Anforderungen einzustellen. Trotz der großen Bedeutung der genannten Verfahren ist es bisher nicht gelungen, Nitrier- und Nitrocarburierprozesse hinreichend genau zu kontrollieren und zu steuern.

Aus der Praxis sind Kontrolleinrichtungen für die Nitrierung bzw. Nitrocarburierung in einem Gasstrom bekannt, die sich auf die Erfassung der Atmosphärenbedingungen bzw. des Atmosphärenzustandes im Behandlungsrezipienten während der Behandlung beschränken. Es kommen im wesentlichen $NH_3$-Analysatoren, aber auch $H_2$-, CO-, $CO_2$-, $CH_4$-Analysatoren zur Erfassung des Atmosphärenzustandes zum Einsatz. Vereinzelt werden auch Massenspektrometer eingesetzt, um die Zusammensetzung der Atmosphäre zu erfassen.

Mittels der aus diesen Messungen ermittelten Nitrierkennzahl wird anhand eines experimentell ermittelten Lehrdiagramms durch die Beeinflussung der Gasdurchflüsse im Behandlungsrezipienten die gewünschte Zusammensetzung der Atmosphäre eingestellt. Es besteht jedoch kein kausaler Zusammenhang zwischen der Zusammensetzung der Atmosphäre und dem Ergebnis der Randschicht-Behandlung, da weitere maßgebliche Einflußgrößen, wie Ofenzustand, Chargengröße, Werkstoff und Werkstoffoberflächenzustand, nicht berücksichtigt werden.

Aus Härterei-technische Mitteilung, Band 41, Nr. 4, Juli/August 1986, R. Konrad et al.: "Zerstörungsfreie Ermittlung von wärmebeeinflussenden Randschichten und deren Dicke", S. 213 bis 218, ist es bekannt, Randschichten nach einer thermochemischen Randschicht-Behandlung zerstörungsfrei zu prüfen.

Die GB-A-2184553 offenbart eine Vorrichtung zur Ermittlung der Phasenumwandlung in warmverformten Metallen und Legierungen.

Aus der EP-A-100 009 ist es bekannt, zerstörungsfrei das Härteprofil einer beispielsweise oberflächengehärteten Werkstoffprobe zu ermitteln. Dazu finden nach der Oberflächenhärtung oder einer sonstigen Wärmebehandlung magnetische Meßmethoden Verwendung. Ähnliches geht aus den Patent Abstracts of Japan, Band 9, Nr. 37 (P-335) (1760) vom 16. Februar 1985 zur JP-A-59178356 der Nippon Denshi K.K. vom 9. Oktober 1984 hervor.

Der genannte Stand der Technik hat den Nachteil, daß er keine Möglichkeit bietet, den Randschichtzustand eines Werkstücks während der Randschicht-Behandlung zu erfassen und daraufhin die Randschicht-Behandlung zu beeinflussen.

Der Erfindung liegt daher die Aufgabe zugrunde, ein Verfahren zur "in situ"-Erfassung und Beeinflussung des Randschichtzustandes von Körpern anzubieten, die einer thermochemischen Randschicht-Behandlung unterzogen werden.

Ein Verfahren zur Lösung dieser Aufgabe weist die Maßnahmen des Anspruchs 1 auf. Das erfindungsgemäße Verfahren bietet den Vorteil, daß im Unterschied zu den bekannten Verfahren nicht eine Ursache der Randschicht-Behandlung, wie z.B. der Atmosphärenzustand, kontrolliert wird, sondern das Ergebnis der Randschicht-Behandlung, nämlich der aktuelle Randschichtzustand erfaßt wird. Durch die Überwachung des Ist-Zustandes der Randschicht wird eine Regelung der Randschicht-Behandlung, insbesondere des Atmosphärenzustandes, zur Erreichung eines vorgegebenen Soll-Zustands der Randschicht möglich. Je nach Einstellung der (Prüf-)Frequenz kann die Messung auf einen bestimmten Randschichtbereich beschränkt werden.

Bei einer thermochemischen (Randschicht-)Behandlung erfolgt die Messung der elektromagnetischen Größe(n) in Abhängigkeit vom Werkstoff des Körpers und der besonderen Art der Behandlung bei einer oder mehreren (Prüf-)Frequenzen. Dieses Verfahren ist besonders geeignet bei einer Randschicht-Behandlung metallischer Werkstoffe durch Nitrieren oder Nitrocarburieren.

Gemessen wird die komplexe elektromagnetische Immitanz, die Auskunft gibt über den elektrischen und magnetischen Zustand des Werkstoffs. Zur Begrenzung des Aufwandes bei der Durchführung des Verfahrens wird die Messung an einer Werkstoffprobe gleichen Werkstoffs, Werkstoffzustandes und Werkstoffrandschichtzustandes wie die übrige, der Randschicht-Behandlung unterzogene Chargierung vorgenommen. Das Verfahren bietet auch die Möglichkeit, die Immitanz einer Kombination des Körpers (Probe) mit elektrotechnischen Bauelementen zu messen.

Die gemessenen Immitanzwerte ermöglichen anhand von Kalibrierkurven den direkten Rückschluß auf den Randschichtzustand kennzeichnende Größen, wie die Einhärtungstiefe, die Diffusionstiefe und die Verbindungs-Schichtdicke.

Durch Abkühlen oder Aufheizen des Körpers (Probe) kann die temperaturabhängige komplexe Immitanz ermittelt werden. Diese führt über die Bestimmung der Phasenumwandlungstemperatur (z.B. Curietemperaturen) zur Ermittlung der Phasenzusammensetzung in der Verbindungsschicht und in der (darunterliegenden) Diffusionsschicht des vermessenen Randschichtbereichs.

Die Größe der Immitanzänderung im Curiepunkt einer Phase ist ein quantitatives Maß für die betreffende Phase. Bei zusammengesetzten Phasen mit konzentrationsabhängigen Curiepunkten, insbesondere bei den ferromagnetischen Phasen $Fe_4N$, $Fe_4$ (C, N), $Fe_{2\text{-}3}$ N,

Fe$_{2-3}$ (C, N), dient die Messung der Immitanzänderung zur Bestimmung der Konzentration von Kohlenstoff und Stickstoff. Die Dicke der aus ferromagnetischen Phasen zusammengesetzten Verbindungsschicht wird aus der Summe der Immitanzänderungen in den Curiepunkten ermittelt.

Das erfindungsgemäße Verfahren ermöglicht eine Steuerung der Randschicht-Behandlung zur Erreichung bestimmter Vorgaben, wie Verbindungsschichtdicke, Einhärtungstiefe, Verbindungsschicht-zusammensetzung und Nitrierhärtetiefe. In Verbindung mit der Atmosphärenregelung im Behandlungsrezipienten ist eine vollautomatische Steuerung und Regelung der Randschicht-Behandlung zur Erreichung eines vorbestimmten Randschichtzustandes durchführbar.

Die Erfindung wird nachfolgend anhand der Zeichnungen näher erläutert. In dieser zeigen:

Fig. 1     einen typischen Widerstandsverlauf bei einer Meßfrequenz von 10 kHz,

Fig. 2     den zusammenhang zwischen Phasenzusammensetzung bzw. Verbindungsschichtdicke und dem relativen Widerstand bei einer Meßfrequenz von 10 kHz,

Fig. 3     eine Abkühlkurve mit Darstellung der temperaturabhängigen Induktivität,

Fig. 4     eine weitere Abkühlkurve mit den Abhängigkeiten gemäß Fig. 3,

Fig. 5     die Abhängigkeit des Curiepunktes der Fe$_{2-3}$N-Phase von der Stickstoffkonzentration, und

Fig. 6     die abhängigkeit des Curiepunktes der Fe$_{2-3}$CN-Phase von der Stickstoff- und Kohlenstoffkonzentration.

Die Erfassung des Randschichtzustandes von Körpern (Werkstoffproben) erfolgt mittels der Messung elektromagnetischer Größen während einer Randschicht-Behandlung, wie z.B. Nitrieren oder Nitrocarburieren, im Behandlungsrezipienten. Demnach erfolgt erfindungsgemäß die Messung "in situ".

Gemessen wird hierbei mit einem Wechselstromsignal (AC). Auf diese Art und Weise kann der von der Meßtechnik her bekannte "Skin-Effekt" ausgenutzt werden, der sich darin äußert, daß sich mit zunehmender Frequenz ein elektromagnetischer Strom auf einen kleiner werdenden Randbereich beschränkt. Somit kann durch Einstellen einer entsprechenden Frequenz der zu vermessende Randbereich variabel eingestellt werden, so daß elektrische und magnetische Eigenschaften von Proben selektiv bis zu sehr dünnen Randochichtbereichen (µm) ermittelt werden können. Der Skin-Effekt hat außerdem zur Folge, daß durch die Verringerung eines stromdurchflossenen Probenquerschnitts das Meßsignal vergrößert wird, und somit eine wesentlich größere Meßempfindlichkeit als bei der Gleichatrommessung erreicht wird.

Mathematisch beschreibt man die Kombination verschiedener elektrischer Leiter als Netzwerksystem. Verwendet man dabei sogenannte Zweipole, wie Widerstände R, Kapazitäten C und Induktivitäten L, aber keine stromerzeugenden Leiter, spricht man von linearen, passiven Netzwerkelementen.

Wird an ein lineares, passives Netzwerksystem eine Wechselspannung U angelegt, fließt ein Strom I. In der komplexen Schreibweise lassen sich beide Größen wie folgt darstellen:

$$U = U_o \cdot e^{jwt}$$

$$I = I_o \cdot e^{j(wt-\theta)}$$

U:      komplexe Wechselspannung
U$_o$:      Effektivspannung
I:      komplexer Wechselstrom
I$_o$:      Effektivstrom
w:      Kreisfrequenz (w = 2πf)
θ:      Phasenwinkel
t:      Zeit

Aus dem Ohmschen Gesetz errechnet sich der komplexe Wechselotromwiderstand, die Impedanz Z, in Abhängigkeit von der Frequenz als Quotient der beiden Größen:

$$Z(w) = U/I = \frac{U_o \cdot e^{jwt}}{I_o \cdot e^{j\,(wt+\theta)}}$$

$$Z(W) = /Z/ \cdot e^{j\theta}$$

Analog zur Impedanz ist der wechselstromleitwert, die Admittanz Y, definiert. Es gilt:

$$Z(w) = 1/Y(w)$$

Impedanz und Admittanz werden unter dem Oberbegriff Immitanz zusammengefaßt.

Im allgemeinen werden die Spannung, der Strom und der Phasenwinkel gemessen und aus diesen Größen werden alle weiteren Folgegrößen errechnet.

Die zur Durchführung des Verfahrens benutzte Meßeinrichtung besteht im wesentlichen aus einem Immitanztäeßgerät, handelsüblich u.a. als LCR-Strom-Spannungs-Meßgerät bezeichnet, einem Meßfühler mit auswechselbarer Meßprobe sowie einer (abgeschirmten) elektrischen Leitung zwischen dem im Behandlungsrezipienten befindlichen Meßfühler und dem Immitanzmeßgerät mit entsprechender Anpassung an den Meßfühler.

Der Meßfühler besteht aus einer temperaturstabilen, chemisch beständigen, elektrischen Durchführung in dem Behandlungsrezipienten mit einer temperaturstabilen, chemisch beständigen elektrischen Isolierung der Meßzuleitungen ($Al_2 O_3$, Glimmer), vobei die Meßzuleitungen mit einer Abschirmung (Ti(C, N) beschichtete ummantelung bzw. Kontaktierungen) versehen ist, um Meßverfälschungen, wie das Driften des Meßsignals, zu vermeiden, und einer elektromagnetischen (induktiven) Kopplung der zuleitungen an die Probe.

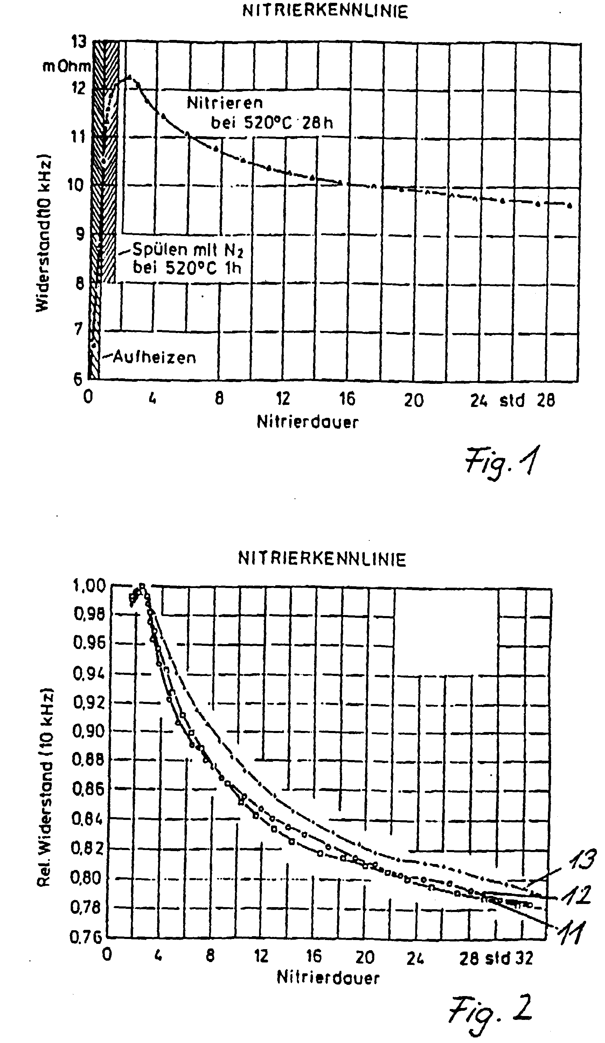

Bei der Nitrierung (32 h) einer Werkstoffprobe aus 31CrMoV9 bei 520 °C und einer Nitrierkennzahl von $k_n$ = 0,4 ergeben sich bei Durchführung des verfahrens mit der eingangs beschriebenen Meßvorrichtung die im folgenden anhand der Fig. 1 bis 6 näher erläuterten Werte.

In Fig. 1 ist ein typischer Widerstandsverlauf bei 10 kHz Meßfrequenz, aus der Immitanzmessung ermittelt, während des gesamten Nitriervorgangs einschließlich Aufheizen und Abkühlen aufgetragen. Hier erkennt man den typisch exponentiellen Verlauf der Meßwerte in Abhängigkeit von der Nitrierdauer, wie er bei allen diffuaionsgesteuerten Prozessen zu erwarten ist.

In Fig. 2 sind drei Messungen an drei vollkommen gleichen probenausgangszuständen während identischer Nitrier-Behandlungen dargestellt. Wie begleitende Untersuchungen zeigten, handelt es sich bei den Proben 11 und 12 sowohl bezüglich der Schichtzusammensetzunq als auch der Dicke der verbindungs- und Diffusionsschicht um identische Randschichtzustände. Dagegen zeigt Probe 13, wie sich selbst geringe Unterschiede in der Phasenzusammensetzung und Dicke der Verbindungsschicht im Meßsignal niederschlagen. Man erkennt, wie empfindlich das Meßsignal den wahren Zustand der Nitrierbehandlung wiederspiegelt.

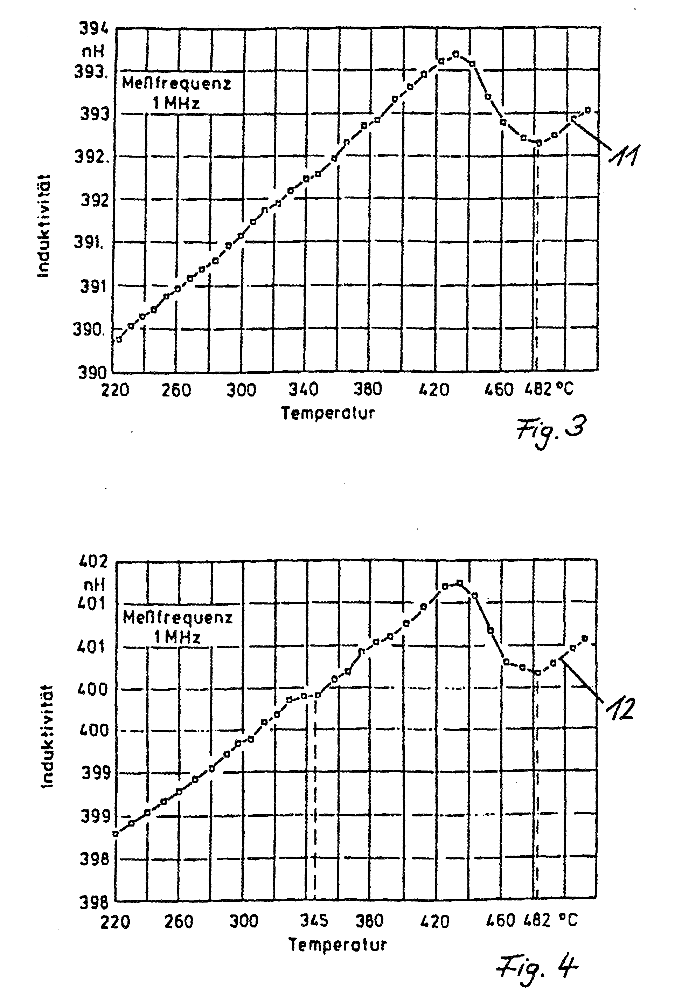

Fig. 3 zeigt eine Abkühlmessung. Dargestellt ist der Induktivitätsverlauf der Meßprobe, aus der Immitanzmessung ermittelt, in Abhängigkeit von der Temperatur. Die Meßfrequenz beträgt 1 MKz. Diese Abkühlkurve wurde am Ende der nitrierbehandlung der Fig. 2 mit der Probe 11 aufgenommen. Sie ist identisch mit dem Verlauf der Meßprobe 12. Bei 482 °C erkennt man deutlich den Curiepunkt der $Fe_4N$-Phase daran, daß die Induktivität trotz fallender Temperatur plötzlich ansteigt bzw. bei geringen Anteilen dieser Phase zumindest einen Haltepunkt aufweist. Der Kurvenverlauf wird parallelversetzt. Die Größe der Parallelverschiebung ist proportional dem Phasenanteil im vermessenen Volumen und kann somit mittels einer Kalibriermessung zur quantitativen phasenbestimmung herangezogen werden.

Die Fig. 4 zeigt die Konzentrationsbestimmung durch die Ermittlung der Curietemperatur(en). Der dargestellte Abkühlkurvenverlauf wurde am Ende der (in Fig. 2 dargestellten) Nitrierbehandlung der Probe 13 aufgenommen. Die Meßfrequenz beträgt 1 MHz. Hier erkennt man einen weiteren Curiepunkt bei 345°C neben dem bei 482°C.

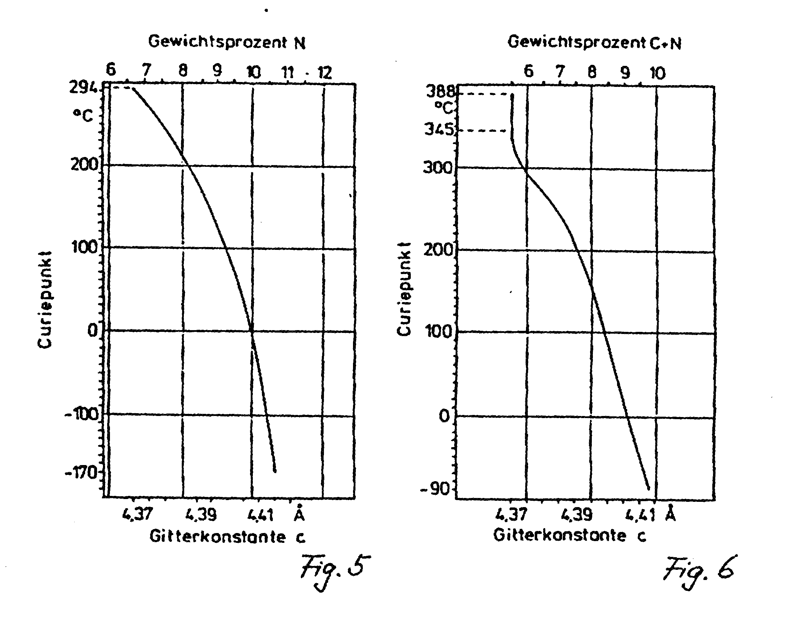

In Fig. 5 ist die Abhängigkeit des Curiepunktes der $Fe_{2-3}N$-Phase von der Stickstoffkonzentration dargestellt. Fig. 6 zeigt die Abhängigkeit des Curiepunktes der $Fe_{2-3}CN$-Phase von der Stickstoff- + Kohlenstoffkonzentration. Die Zuordnung ergibt hier eine zusätzliche $\varepsilon$-Eisen-Carbonitridphase mit der Konzentration von 5,5 Gew.%.

## Patentansprüche

1. Verfahren zur Erfassung des Randschichtzustandes von Körpern aus metallischem Werkstoff, bei welchem eine vom Körper abhängige Immitanz des Körpers, wobei Impedanz und Admittanz unter dem Begriff Immitanz zusammengefaßt sind, oder die Verstimmung eines Schwingkreises, dessen Bestandteil der Körper ist, während einer thermochemischen Randschicht-Behandlung fortlaufend gemessen und die dabei ermittelten Immitanzwerte bzw. Verstimmungswerte mit einem einem vorgegebenen Soll-Zustand der Randschicht entsprechenden Immitanzwert bzw. Verstimmungswert verglichen werden, und bei welchem mit einer Frequenz gemessen wird, die derart eingestellt wird, daß durch Skin-Effekt nur ein Randbereich des Körpers erfaßt wird, der sich mindestens über den Bereich der Randschicht-Behandlung erstreckt.

2. Verfahren nach Anspruch 1, dadurch gekennzeichnet, daß in Abhängigkeit vom Werkstoff des Körpers und von der Art der Behandlung die Messung der Immitanz bei einer oder mehreren Frequenzen erfolgt.

3. Verfahren nach Anspruch 1 oder 2, dadurch gekennzeichnet, daß an dem Körper die komplexe elektromagnetische Immitanz im Behandlungsrezipienten ganz oder teilweise (z.B. Phasenwinkel, Widerstand, Induktivität) unterhalb der magnetischen Sättigung gemessen wird.

4. Verfahren nach Anspruch 1 sowie einem oder mehreren der weiteren Ansprüche, dadurch gekennzeichnet, daß die Immitanz einer Kombination des Körpers mit elektromagnetischen Bauelementen gemessen wird.

5. Verfahren nach Anspruch 1 sowie einem oder mehreren der weiteren Ansprüche, dadurch gekennzeichnet, daß die Einhär-tungstiefe, die Diffusionstiefe und die (Verbindungs-) Schichtdicke im Bereich der Randschicht des Körpers (Probe) anhand von Kalibrierkurven aus den gemessenen Immitanzwerten ermittelt werden.

6. Verfahren nach Anspruch 1 sowie einem oder mehreren der weiteren Ansprüche, dadurch gekennzeichnet, daß Phasenumwandlungen durch Messung der temperaturabhängigen, komplexen elektromagnetischen Immitanz, Abkühlen oder Aufheizen des Körpers im Behandlungsrezipienten ermittelt werden.

7. Verfahren nach Anspruch 1 sowie einem oder mehreren der weiteren Ansprüche, dadurch gekennzeichnet, daß aus der Bestimmung der Curiepunkte mittels temperaturabhängiger ImmitanzMessung im vermessenen Randschicht-Bereich, insbesondere in der Verbindungsschicht des nitrierten bzw. nitrocarburierten Körpers (Probe), die ferromagnetischen Phasen ermittelt werden zur Bestimmung der Existenz dieser Phasen im Körper (Probe).

8. Verfahren nach Anspruch 1 sowie einem oder mehreren der weiteren Ansprüche, dadurch gekennzeichnet, daß die Größe der Immitanzänderung im Curiepunkt einer Phase ein quantitatives Maß für die betreffende Phase darstellt.

9. Verfahren nach Anspruch 1 sowie einem oder mehreren der weiteren Ansprüche, dadurch gekennzeichnet, daß bei allen Phasen, deren Curiepunkte konzentrationsabhängig sind, insbesondere bei den ferromagnetischen Phasen $Fe_4\,N$, $Fe_4\,(C, N)$, $Fe_{2\text{-}3}\,N$, $Fe_{2\text{-}3}\,(C, N)$, die exakte Ermittlung der Curietemperatur, die Konzentration des Kohlenstoffs und Stickstoffs bestimmt werden.

10. Verfahren nach Anspruch 1 sowie einem oder mehreren der weiteren Ansprüche, dadurch gekennzeichnet, daß die Dicke der aus ferromagnetischen Phasen zusammengesetzten Verbindungsschicht durch die Summe der Immitanzänderungen in den Curiepunkten ermittelt wird.

11. Verfahren nach Anspruch 1 sowie einem oder mehreren der weiteren Ansprüche, dadurch gekennzeichnet, daß es zur Steuerung thermochemischer Randschicht-Behandlungen von Körpern (Proben) eingesetzt wird, insbesondere im Hinblick auf Verbindungsschichtdicke, Einhärtungstiefe, Verbindungs-Schichtzusammensetzung und Nitrierhärtetiefe.

12. Verfahren nach Anspruch 1 sowie einem oder mehreren der weiteren Ansprüche, dadurch gekennzeichnet, daß in Verbindung mit einer Atmosphärenregelung eine vollautomatische Steuerung und Regelung der thermochemischen Randschicht-Behandlung erfolgt, derart, daß vorgegebene, definierte Behandlungsziele reproduzierbar sind.

13. Verwendung des Verfahrens nach den Ansprüchen 1 bis 12 zur Erfassung des Randschicht-Zustandes von Körpern bei einer Randschicht-Behandlung.

14. Verwendung des Verfahrens nach den Ansprüchen 1 bis 12 zur Erfassung des Randschichtzustandes von Körpern beim Nitrieren/Nitrocarburieren.

## Claims

1. Method for recording the surface layer state of bodies made of metallic material, wherein an immittance of the body, said immittance depending on the body, impedance and admittance being conjoined under the term immittance, or the off-resonance state of an oscillating circuit of which the body is a component are continuously measured during a thermochemical surface layer treatment, and the immittance values or off-resonance values determined in the process are compared with an immittance value or off-resonance value corresponding to a predetermined nominal state of the surface layer, and wherein measurements are carried out with a frequency which is adjusted in such a way that, owing to a skin effect, only a surface region of the body is measured, which extends at least over the domain of the surface layer treatment.

2. Method according to Claim 1, characterized in that, depending on the material of the body and on the type of the treatment, the measurement of the immittance takes place at one or more frequencies.

3. Method according to Claim 1 or 2, characterized in that measurement, on the body, of the complex electromagnetic immittance in the treatment receiver takes place entirely or in part (e.g. phase angle, resistance, inductivity) below magnetic saturation.

4. Method according to Claim 1 and one or more of the further claims, characterized in that the immittance of a combination of the body with electromagnetic components is measured.

5. Method according to Claim 1 and one or more of the further claims, characterized in that the hardness penetration depth, the diffusion depth and the (compound) layer thickness in the region of the surface layer of the body (sample) are determined from the measured immittance values on the basis of calibration curves.

6. Method according to Claim 1 and one or more of the further claims, characterized in that phase transitions are determined by measuring the temperaturedependent complex electromagnetic immittance, cooling or heating of the body in the treatment receiver.

7. Method according to Claim 1 and one or more of the further claims, characterized in that, based on the determination of the Curie points by means of temperature-dependent immittance measurement in the surface layer region measured, in particular in the compound layer of the nitrided or nitrocarburized body (sample), the ferromagnetic phases are determined for the purpose of establishing the existence of these phases in the body (sample) .

**8.** Method according to Claim 1 and one or more of the further claims, characterized in that the magnitude of the immittance change at the Curie point of a phase represents a quantitative measure for the phase in question.

**9.** Method according to Claim 1 and one or more of the further claims, characterized in that the exact determination of the Curie temperature, the concentration of the carbon and nitrogen are determined for all phases whose Curie points are concentration-dependent, in particular for the ferromagnetic phases $Fe_4 N$, $Fe_4 (C, N)$, $Fe_{2-3} N$, $Fe_{2-3} (C, N)$.

**10.** Method according to Claim 1 and one or more of the further claims, characterized in that the thickness of the compound layer composed of ferromagnetic phases is determined by the sum of the immittance changes at the Curie points.

**11.** Method according to Claim 1 and one or more of the further claims, characterized in that it is employed to control thermochemical surface layer treatments of bodies (samples), in particular with respect to compound layer thickness, hardness penetration depth, compound layer composition and nitriding hardness depth.

**12.** Method according to Claim 1 and one or more of the further claims, characterized in that fully automatic regulation and control of the thermochemical surface layer treatment takes place, in conjunction with the atmosphere being controlled, in such a way that predetermined defined treatment objectives are reproducible.

**13.** Use of the method according to Claims 1 to 12 for recording the surface layer state of bodies in the context of a surface layer treatment.

**14.** Use of the method according to Claims 1 to 12 for recording the surface layer state of bodies in the context of nitriding/nitrocarburizing.

**Revendications**

**1.** Procédé de détermination de l'état de la couche superficielle de corps métalliques, dans lequel on mesure de façon continue, pendant un traitement thermochimique de cette couche superficielle, une "immitance" du corps dépendant de celui-ci, l'impédance et l'admittance étant réunies sous le terme générique d'"immitance", ou le désaccord d'un circuit oscillant dont le corps fait partie, et on compare les valeurs d'"immitance" ou de désaccord obtenues avec une valeur d'"immitance" ou de désaccord correspondant à un état prescrit fixé de la couche superficielle, et dans lequel on fait la mesure à une fréquence choisie de façon telle qu'on ne saisisse par effet de peau qu'une zone superficielle du corps qui s'étend au moins sur la zone de traitement de la couche superficielle.

**2.** Procédé selon la revendication 1, caractérisé par le fait que, suivant le matériau du corps et la nature du traitement, on mesure l'"immitance" à une ou plusieurs fréquences.

**3.** Procédé selon l'une des revendications 1 et 2, caractérisé par le fait qu'on mesure sur le corps l'"immitance" électromagnétique complexe dans le récipient de traitement entièrement ou partiellement (par exemple angle de phase, résistance, inductance) au-dessous de la saturation magnétique.

**4.** Procédé selon la revendication 1 et une ou plusieurs des autres revendications, caractérisé par le fait qu'on mesure l'"immitance" d'une combinaison du corps avec des composants électromagnétiques.

**5.** Procédé selon la revendication 1 et une ou plusieurs des autres revendications, caractérisé par le fait qu'on détermine la profondeur de trempe, la profondeur de diffusion et l'épaisseur de la couche (de liaison) dans la zone de la couche superficielle du corps (échantillon) à l'aide de courbes d'étalonnage à partir des valeurs d'"immitance" mesurées.

**6.** Procédé selon la revendication 1 et une ou plusieurs des autres revendications, caractérisé par le fait qu'on détermine des changements de phase par mesure de l'"immitance" électromagnétique complexe dépendant de la température, refroidissement ou chauffage du corps dans le récipient de traitement.

**7.** Procédé selon la revendication 1 et une ou plusieurs des autres revendications, caractérisé par le fait qu'à partir de la détermination des points de Curie au moyen de mesure d'"immitance" dépendant de la température dans la zone mesurée de la couche superficielle, en particulier dans la couche de liaison du corps (échantillon) nitruré ou carbonitruré, on détermine les phases ferromagnétiques pour déterminer l'existence de ces phases dans le corps (échantillon).

**8.** Procédé selon la revendication 1 et une ou plusieurs des autres revendications, caractérisé par le fait que la grandeur de la variation de l'"immitance" au point de Curie d'une phase constitue une mesure quantitative de la phase considérée.

9. Procédé selon la revendication 1 et une ou plusieurs des autres revendications, caractérisé par le fait que pour toutes les phases dont les points de Curie dépendent de la concentration, en particulier les phases ferromagnétiques $Fe_4N$, $Fe_4(C, N)$, $Fe_{2-3}N$, $Fe_{2-3}(C, N)$, on détermine exactement la température de Curie et on détermine la concentration de carbone et d'azote.

10. Procédé selon la revendication 1 et une ou plusieurs des autres revendications, caractérisé par le fait qu'on détermine l'épaisseur de la couche de liaison composée de phases ferromagnétiques par la somme des variations de l'"immitance" aux points de Curie.

11. Procédé selon la revendication 1 et une ou plusieurs des autres revendications, caractérisé par le fait qu'on l'utilise pour la commande de traitements thermochimiques de la couche superficielle de corps (échantillons), en particulier en considération de l'épaisseur de la couche de liaison, de la profondeur de trempe, de la composition de la couche de liaison et de la profondeur de dureté de nitruration.

12. Procédé selon la revendication 1 et une ou plusieurs des autres revendications, caractérisé par le fait qu'en association avec une régulation de l'atmosphère ont lieu une commande et une régulation entièrement automatiques du traitement thermochimique de la couche superficielle de façon telle que des objectifs de traitement définis prescrits soient reproductibles.

13. Utilisation du procédé selon les revendications 1 à 12 pour la détermination de l'état de la couche superficielle de corps lors d'un traitement de cette couche.

14. Utilisation du procédé selon les revendications 1 à 12 pour la détermination de l'état de la couche superficielle de corps lors de la nitruration et de la carbonitruration.

NITRIERKENNLINIE

Fig. 1

NITRIERKENNLINIE

Fig. 2

EP 0 324 809 B1

Fig. 3

Fig. 4

Fig. 6

Fig. 5